# EUROPEAN PATENT SPECIFICATION

(11) **EP 1 082 126 B1**
(45) Date of publication and mention of the grant of the patent: **13.02.2008**
(21) Application number: 99925818.9
(22) Date of filing: 26.05.1999
(51) Int. Cl.: A61K 38/00, A61K 38/04, C07K 5/00, C07K 7/00, C12N 5/00

(54) **NOVEL PEPTIDES**
NEUE PEPTIDE
NOUVEAUX PEPTIDES

(30) Priority: 27.05.1998 US 84371
(43) Date of publication of application: 14.03.2001
(73) Proprietor: HADASIT MEDICAL RESEARCH SERVICES & DEVELOPMENT CO. LTD., Jerusalem 91120 (IL); V.I. Technologies, Inc., New York, NY 10032 (US)
(72) Inventor: GORODETSKY, Raphael, 96951 Jerusalem (IL); MARX, Gerard, New York, NY 10033 (US)
(74) Representative: Brown, David Leslie
(86) International application number: PCT/US1999/011517
(87) International publication number: WO 1999/061041

(56) References cited:
- US-A- 4 455 290
- US-A- 5 292 362
- US-A- 5 428 014
- US-A- 5 473 051
- US-A- 5 599 790
- US-A- 5 639 940
- PHILLIPS D R ET AL: "THE PLATELET MEMBRANE GLYCOPROTEIN IIB-IIIA COMPLEX" BLOOD, vol. 71, no. 4, 1988, pages 831-843, XP002218130 ISSN: 0006-4971
- NIEMAN C J ET AL: "A COLOURMETRIC ENZYME-LINKED SANDWICH ASSAY FOR THE DETECTION OF HUMAN PLATELETS BOUND TO A FIBRINOGEN-COATED SURFACE" THROMBOSIS RESEARCH, TARRYTOWN, NY, US, vol. 62, no. 3, 1991, pages 189-197, XP001118065 ISSN: 0049-3848
- FU Y ET AL: "Fibrinogen alpha genes: Conservation of bipartite transcripts and carboxy-terminal-extended alpha subunits in vertebrates" GENOMICS, ACADEMIC PRESS, SAN DIEGO, US, vol. 30, no. 1, 1995, pages 71-76, XP002218053 ISSN: 0888-7543
- THOMPSON W D ET AL: "ANGIOGENIC ACTIVITY OF FIBRIN DEGRADATION PRODUCTS IS LOCATED IN FIBRIN FRAGMENT E" JOURNAL OF PATHOLOGY, CHICHESTER, SUSSEX, GB, vol. 168, no. 1, September 1992 (1992-09), pages 47-53, XP000983610 ISSN: 0022-3417
- CHUNG et al., "Characterization of Complementary Deoxyribonucleic Acid and Genomic Deoxyribonucleic Acid for the beta Chain of Human Fibrinogen", BIOCHEMISTRY, 1983, Vol. 22, No. 13, pages 3244-3250,. XP002914827.
- BLUMENSTEIN et al., "A beta-Turn is Present in the 392-411 Segment of the Human Fibrinogen gamma-Chain. Effects of Structural Changes in This Segment on Affinity to Antibody 4A5", BIOCHEMISTRY, 1992, Vol. 31, No. 44, pages 10692-10698, XP002923721.
- FU et al., "Carboxy-Terminal-Extended Variant of the Human Fibrinogen alpha Subunit: A Novel Exon Conferring Marked Homology to beta and gamma Subunits", BIOCHEMISTRY, 1992, Vol. 31, No. 48, pages 11968-11972, XP002923722.

## Description

### FIELD AND BACKGROUND

The present invention relates to novel peptides, and in particular, to novel peptides which are homologous to a portion of the carboxy termini of fibrinogen, as well as to potential uses for these peptides.

Fibrinogen is the plasma protein which forms the clot when blood coagulates. Many studies have been conducted on the amino acid sequences and structure of fibrinogen (Mosesson, M. and Doolittle, R. (Eds.) "The biology of fibrinogen and fibrin", Ann. N.Y. Acad. Sci., 408, 1983, Henschen, A. et al., "Structure of fibrinogen", Ann. N.Y. Acad. Sci., 408, 1983, Spraggon, G. et al., "Crystal structure of fragment D from human fibrinogen and its crosslinked counterpart from fibrin", Nature, 389:455-462, 1997, Murakawa, M. et al., "Diversity of primary structures of the carboxy-terminal regions of mammalian fibrinogen Aa-chains", Thromb. & Haemostat., 69:351-360, 1993). Normally, fibrinogen itself has a molecular weight of 340 kDa and is constructed from two sets of three peptide chains, named α, β and γ. The constituent chains of fibrinogen are highly conserved between species. Recent work has also described a fibrinogen protein with a longer α chain, called αE fibrinogen, which has a concomitantly higher molecular weight of 420 kDa and which may play a role in development (Fu, Y. and Grieninger, G. "Fib420 : A normal human variant of fibrinogen with two extended a chains", Proc.Natl.Acad.Sci.USA, 91: 2625-2628, (1994), Fu, Y, et al., "Carboxy-terminal-extended variant of the human fibrinogen α subunit: A novel exon conferring marked homology to β and γ subunits" Biochem., 31:11968-11972, (1992)). Thus, these four types of fibrinogen chains, α, β, γ and αE, each with 610, 410, 391 and 1096 amino acids, respectively.

Fibrin clots are formed *in vivo* at the sites of tissue injury based upon the reaction of fibrinogen and thrombin in the presence of calcium ions. These clots have a major role in hemostasis. After clot formation, fibrin serves a provisional matrix for cell recruitment into the wound bed. Normally, the earliest cells mobilized into the wound bed are inflammatory, such as leukocytes and particularly macrophages. Concomitant with their penetration into the fibrin, these inflammatory cells participate in lysing the fibrin by generating plasmin, metallo-proteinases (MIPs) and/or free radicals. Thus, the wound bed contains substantial quantities of peptides A and B (FPA and FPB) released by thrombin during the onset of coagulation, followed by numerous fibrin breakdown products generated by lytic enzymes or free-radicals (Gray, A.J., Reeves, J.T., Harrison, N.K., Winlove, P. and Laurent, G.J., "Growth factors for human fibroblasts in the solute remaining after clot formation", J.Cell Sci., 96: 271-274, (1990), Marx G. "Immunological monitoring of Fenton fragmentation of fibrinogen", Free Radicals Res. Comm. 12: 517-520 (1991), Francis, C.W., Marder, V.J. and Barlow, G.H., "Plasmic degradation of crosslinked fibrin", J.Clin Invest., 66: 1033-1043, (1980), Cottrell, B. A.and Doolittle, R.F."The amino acid sequence of a 27-residue peptide released from α-chain carboxy-terminus during the plasmic digestion of human fibrinogen", Acad.Press., 71: 754-766, (1976)).

Subsequently, the inflammatory cells are followed by the migration of cells of the mesenchyme lineage such as fibroblasts which further digest fibrin, replacing it with extracellular matrix (ECM). Endothelial cells also infiltrate the wound bed and generate microcapillary structures. Ultimately these cells replace the provisional fibrin matrix with granulation tissue populated by parenchymal cells and vasculature within ECM.

The attachment and migratory responses of cells are controlled by specific receptors (integrins) or by intercellular adhesion molecules (ICAM) that interact with cell membrane receptors which trigger either migratory reactions or cell adhesion to matrix. These interactions may trigger other regulatory mechanisms of cell activity, migration or proliferation. Growth factors and cytokines activate these cell receptors by binding to them, and thus, trigger these cellular responses.

Cytokines of different classes regulate cellular activity and responses, control cell survival, growth and differentiation. Excluding classical endocrine hormones, cytokines encompass those families of cell regulators variously known as growth factors, interleukins, lymphokines and interferons.

All previously described cytokines are composed of more than 50 amino acids (aa); most are over 100 aa long. Based on X-ray crystallography, cytokines exhibit 8 structural groups (Nathan C. & Sporn M., "Cytokines in context", J. Cell. Biol.113: 981-986 (1991)) and bind to a variety of cellular receptors such as integrins or interferon receptors. Binding to cell receptors triggers a cascade of events leading to intra-cellular phosphorylation of proteins, which is transduced into gene expression, cell proliferation, cell differentiation, changes in cell shape, motility and apoptosis. Thus, cytokines play an important role in physiological processes such as development and wound healing.

Human fibroblasts are the major cellular entities responsible for the regeneration of the extracellular matrix within the wound bed. Human fibroblasts also express specific membrane receptors to fibrinogen and thrombin. In the case of skin wounds, human fibroblasts reform the matrix of the dermis. For example, during the course of healing of an incisional skin wound, human fibroblasts are mobilized from the surrounding tissue and enter into the fibrin clot, help to dissolve the clot, and then generate as well as reform the collagens in the extracellular matrix. Based upon these properties of human fibroblasts, fibroblast implants have been suggested to supplement the process of healing in damaged skin (Gorodetsky, R. et al., Radiat. Res., 125:181-186, 1991).

One example of this approach is the use of benzoylated hyaluronic acid (HA) sheets containing holes or pores as a carrier for fibroblasts and keratinocytes for wound healing (Andreassi, L., et al., Wounds, 3:116-126, 1991). Specifically, HA sheets were cultured with such cells which grow within the pore structure. The sheets were then affixed to the site of the burn injury, where the cells migrated out of the sheet and ultimately accelerated the rate of wound re-granulation. A major problem with implanted HA sheets, however, is that they are not metabolized by tissue, are mechanically cumbersome to administer, and may cause undesired immunological effects in the long term.

The inventors of the present invention have previously found that fibrin microbeads (FMB) possess both chemotactic and proliferative effects for certain types of cells. These cells include fibroblasts and smooth muscle endothelial cells, but typically not keratinocytes. The cells were shown to migrate into these beads by chemotaxis, and then to proliferate within the beads. Furthermore, the cells were shown to remain stable for prolonged periods of time when cultured within the fibrin microbeads. Thus, the fibrin microbeads appeared to stimulate both cell chemotaxis and cell growth.

However, the fibrin microbeads themselves have certain inherent limitations because of their structure. In other words, the fibrin microbeads are particularly useful only as three-dimensional structures of cross-linked fibrin(ogen). If other structures were desired, and in particular if the lack of such was desired, fibrin microbeads would not be particularly useful. Furthermore, fibrin microbeads would not be particularly useful for avoiding the use of plasma proteins.

A more useful approach would identify the smallest components of fibrin(ogen) responsible for the desired chemotactic and haptotaxis properties. Attempts have been made to find these small components within the larger fibrin(ogen) molecule. A voluminous literature exists which describes the binding of fibrinogen (γ 400-411) to platelets through the GPIIb/IIIa receptor (see for example Savage B., Bottini E. & Ruggeri ZM., "Interaction ofintegrin alpha IIb beta with multiple fibrinogen domains during platelet adhesion", J. Biol. Chem. 270: 28812-7 (1995)), and the aggregation activity of the amino Bβ 15-42 terminus which is exposed after release of fibrinopeptide B. In addition, a peptide containing the 16 amino acids of the sequence of the g-carboxy terminus of fibrinogen was synthesized and was found to bind to platelet integrin (D'Souza, S.E. et al., J. Biol. Chem., 265:3440-3446, 1990). However, the biological activities of only a few other fibrinogen breakdown products have been investigated. The activity of these different breakdown products seems to be widely variable.

For example, fragment E was reported to exhibit angiogenic properties and to inhibit endothelial cell migration in a Boyden chamber chemotactic assay (Thompson, W.D., Smith , E.B., Stirk, C.M., Marshall, F.I., Stout, A.J.and Kocchar, A., "Angiogenic activity of fibrin degradation products is located in fibrin fragment E", J.Pathol., 168: 47-53 (1992)). Fragment D was reported to cause detachment of cultured endothelial cells from the extracellular matrix (ECM) substratum in a process which was both concentration and time dependent (Savage B., Bottini E. & Ruggeri ZM., "Interaction of integrin alpha IIb beta with multiple fibrinogen domains during platelet adhesion", J. Biol. Chem. 270: 28812-7 (1995)). Isolated constituent chains of fibrinogen (Aα1, Aα2 and Bβ) released upon activation of the fibrinogen by thrombin were observed to stimulate fibroblast proliferation by 23-31 % above controls, whereas isolated γ chain had no effect (Gray, A.J., Bishop, J.E., Reeves, J.T. and Laurent, G.J.; "Aa and Bβ Chains of fibrinogen stimulate proliferation of human fibroblasts", J. Cell Sci., 104: 409-413, (1993)). Human polymorphonuclear leukocytes (PMN) were shown to bind to fibrin(ogen) coated surfaces via a type 3 (CD11b/CD18) complement receptor homologous to the GPIIb/IIIa receptor through a decamer of the γ chain carboxy terminus (LGGAKQAGDV). Vasoactive peptides corresponding to residues 43-47 of the Bβ chain and 220-230 of the Aα chain were identified (Gray, A.J., Bishop, J.E., Reeves, J.T. and Laurent, G.J.; "Aa and Bβ Chains of fibrinogen stimulate proliferation of human fibroblasts", J. Cell Sci., 104: 409-413, (1993)).

Fibrinogen itself, when bound to sepharose beads, did not significantly affect cell proliferation, but exerted haplotactic/adhesive activity with human (HF) or mouse (MF) fibroblasts, endothelial cells (EC) and smooth muscle (SMC) cells. Thrombin treatment of fibrinogen-sepharose beads (FB), which would not affect the carboxy terminals of the molecule, did not alter cellular responses, though plasmin, which clips off gamma carboxy termini and digests D-domain sequences, nearly totally abrogated the cell-attractant properties of FB.

Certain portions of the fibrin molecule have been hypothesized to express cell binding properties. However, the identity of all segments responsible for the chemotactic and cell proliferative effects of fibrin(ogen) has not been specified. The identification of such segments would enable more specific intervention in the wound healing process and in the development of novel therapeutic compositions or devices. Furthermore, novel diagnostic tests could potentially be developed, based upon the response, or lack thereof, of cells to the effects of such segments, for example. Thus, the identification of these specific segments or peptides exhibiting cellular activity would have great utility.

There is thus a recognized need for, and it would be highly advantageous to have, a peptide or peptides with specifically determined cellular effects, such as cell proliferative and chemotactic properties, which do not require the presence of the entirety of the fibrin molecule to exert cellular effects.

### SUMMARY OF THE INVENTION

It is one object of the present invention to provide peptides with novel amino acid sequences which are featured within the carboxy termini of fibrinogen.

It is another object of the present invention to provide such peptides useful for pharmaceutical compositions.

It is still another object of the present invention to provide such peptides useful for cell culture and cell separation.

It is yet another object of the present invention to provide such peptides useful for novel cell structures, including biomedical devices.

These and other objects of the present invention are explained in greater detail in the description, Figures and claims below.

The novel peptide sequences of the present invention are homologous to regions of the fibrin molecule, yet retain certain desired properties of the entire molecule, such as cell adhesive effects, for example. These peptides are KGSWYSMRKMSMKIRPFFPQQ (peptide-09), and haptotactic fragments thereof.

According to the teachings of the present invention, there is provided a peptide, derived from fibrinogen, consisting of a chain of amino acids having a sequence of KGSWYSMRKMSMKIRPFFPQQ or a haptotactic fragment thereof.

According to another embodiment of the present invention, there is provided a composition, comprising the said peptide. Preferably, the composition further comprises a pharmaceutically acceptable carrier. Also preferably, the composition further comprises a biological agent selected from the group consisting of drugs, vitamins, vitamin derivatives, growth factors, glucocorticosteroids, steroids, antibiotics, toxins, enzymes, enzyme inhibitors, immunomodulators, immunoglobulins and fragments thereof, fatty acid derivatives, polysaccharides, cell receptor binding molecules, anti-inflammatories, nucleic acids, and polynucleotides.

According to preferred embodiments of the present invention, the composition further comprises a cell selected from the group consisting of fibroblasts, endothelial cells, chondrocytes, neuroblastoma cells, kidney cells, liver cells, pancreatic cells, thyroid cells, glial cells, smooth muscle cells, mouse mammary carcinoma cells, bone or cartilage forming cells, and combinations thereof. Preferably, the cell type is selected from the group consisting of fibroblasts, smooth muscle cells, endothelial cells, chondrocytes, and combinations thereof. More preferably, the cell type is a fibroblast.

The present invention enables a method for promoting healing of a wound in a subject, the method comprising the steps of: (a) providing a composition, the composition featuring the said peptide; and (b) administering a pharmaceutically effective amount of the composition to the wound of the subject. Preferably, the composition further includes a biological agent, the biological agent being selected from the group consisting of drugs, vitamins, vitamin derivatives, growth factors, glucocorticosteroids, steroids, antibiotics, toxins, enzymes, enzyme inhibitors, immunomodulators, immunoglobulins and fragments thereof, fatty acid derivatives, polysaccharides, cell receptor binding molecules, anti-inflammatories, nucleic acids, and polynucleotides.

Also preferably, the composition further includes a cell selected from the group consisting of fibroblasts, endothelial cells, chondrocytes, neuroblastoma cells, kidney cells, liver cells, pancreatic cells, thyroid cells, glial cells, smooth muscle cells, mouse mammary carcinoma cells, bone or cartilage forming cells, and combinations thereof.

According to another embodiment of the present invention, there is provided the use of the said composition for the manufacture of a medicament for promoting healing of a wound in a subject.

According to yet another embodiment of the present invention, there is provided a cell structure, comprising: (a) the said peptide (b) a cell bound to the peptide; and (c) a structure for supporting the cell, the peptide being attached to the structure such that the cell is supported by the structure. Preferably, the structure is a biomedical device. More preferably, the biomedical device is a prosthesis. Also more preferably, the structure is a gel. Alternatively and more preferably, the structure is a sheet of collagen.

According to another embodiment of the present invention, there is provided a composition for assessing binding to a receptor, comprising: (a) the said peptide; and (b) a reporter for transmitting a signal, wherein an ability of the peptide to bind to the receptor is determined according to the signal transmitted by the reporter. Preferably, the reporter is selected from the group consisting of a fluorescent moiety and a radioactive moiety. Also preferably, the receptor is a protein. Also preferably, the receptor is an integral part of a cell.

According to yet another embodiment of the present invention, there is provided a method for determining binding of a peptide to a receptor, the method comprising the steps of: (a) providing the said peptide labeled with a reporter for transmitting a signal; (b) contacting the peptide with the receptor; and (c) determining binding of the peptide to the receptor according to the signal transmitted by the reporter.

According to still another embodiment of the present invention, there is provided a method for separating a cell from a mixture, the cell being capable of binding to a peptide, the method comprising the steps of: (a) incubating the mixture with the said peptide, such that the cell binds to the peptide; and (b) removing the peptide from the mixture, such that the cell is separated from the mixture. Preferably, the peptide is attached to a support, such that the step of removing the peptide is performed by separating the support and the mixture. More preferably, the support is a gel chromatography matrix.

According to yet another embodiment, there is provided a system for culturing of a cell capable of binding to a peptide, comprising: (a) a structure for attaching the said peptide, such that the cell binds to the peptide and grows on the structure; and (b) a culture medium for contacting the cell to provide nourishment to the cell.

According to another embodiment of the present invention, there is provided a polymer, comprising: (a) a plurality of subunits, each subunit featuring at least one said peptide; and (b) a plurality of linker moieties for attaching each of the plurality subunits to another of the plurality of subunits to form the polymer. Preferably, the subunit is comprised of the at least one peptide, such that the polymer is a peptide polymer. Alternatively and preferably, the at least one peptide is attached to the subunit, such that the polymer is a co-polymer.

Hereinafter, the term "wound-healing cells" refers to those cells which promote healing of a wound, including, but not limited to, fibroblasts, smooth muscle endothelial cells and keratinocytes.

The term "fibrin(ogen)" is known in the art as a mixture of fibrin and fibrinogen, and is referred to herein according to this definition. Hereinafter, the term "biologically active" refers to molecules, or complexes thereof, which are capable of exerting an effect in a biological system. Hereinafter, the term "fragment" refers to a portion of a molecule or a complex thereof, in which the portion includes substantially less than the entirety of the molecule or the complex thereof.

Hereinafter, the term "amino acid" refers to both natural and synthetic molecules which are capable of forming a peptidic bond with another such molecule. Hereinafter, the term "natural amino acid" refers to all naturally occurring amino acids, including both regular and non-regular natural amino acids. Hereinafter, the term "regular natural amino acid" refers to those amino acids which are normally used as components of a protein. Hereinafter, the term "non-regular natural amino acid" refers to naturally occurring amino acids, produced by mammalian or non-mammalian eukaryotes, or by prokaryotes, which are not usually used as a component of a protein by eukaryotes or prokaryotes. Hereinafter, the term "synthetic amino acid" refers to all molecules which are artificially produced and which do not occur naturally in eukaryotes or prokaryotes, but which fulfill the required characteristics of an amino acid as defined above. Hereinafter, the term "peptide" includes both a chain of a sequence of amino acids, and analogues and mimetics having substantially similar or identical functionality thereof, including analogues having synthetic and natural amino acids. As shown in Table 1 below, peptide-07 has the amino acid sequence of the C-terminus of the alpha chain of fibrinogen.

### BRIEF DESCRIPTION OF THE DRAWINGS

The invention is herein described, by way of example only, with reference to the accompanying drawings, wherein:
FIG. 1 shows the lack of proliferative effect by the peptides of the present invention on mouse fibroblasts (MF), with Figure 1A showing the lack of effect of peptide-07, Figure 1B for peptide-09, Figure 1C for peptide-71 and Figure 1D for peptide-70;
FIG. 2 shows the haptotactic effect (attachment) of the peptides of the present invention covalently bound to sepharose beads (SB) for various cell types, with Figure 2A showing the degree of attachment of SB-07, SB-09, SB-fibronectin or SB-albumin on mouse and human fibroblasts, Figure 2B showing the haptotactic effect of SB-07, SB-09, SB-fibronectin or SB-albumin on smooth muscle cells, and Figure 2C shows the haptotactic effect of SB-70 or SB-71 on smooth muscle cells and human fibroblasts;
FIG. 3 shows the percentage attachment of various cell types tested to SB-peptides of the present invention;
FIG. 4 shows FITC-labeled peptide-71 binding and uptake by human fibroblasts, at one hour (Figures 4A and 4B) and two hours of incubation (Figures 4C-E), as well as concentration dependence of uptake for FITC-labeled peptide-09 for EC cells; and
FIG. 5 shows FACS analysis of FITC-labeled peptides-09 (Figures 5A-5C), 70 (Figure 5E), 71 (Figure 5D) and 07 (Figure 5F).

### BRIEF DESCRIPTION OF THE INVENTION

The present invention relates to novel peptide and nucleotide sequences, and in particular, to novel peptide amino acid sequences and equivalent nucleotide sequences, as well as to potential uses for these sequences. For example, these peptide sequences have potential medical uses, such as for therapeutic and diagnostic uses. The peptide sequences are homologous to regions of the fibrin molecule, yet retain certain desired properties of the entire molecule, such as cell adhesive effects, for example.

In particular, these peptides are carboxy-terminal fibrinogen sequences, especially the terminal 20 amino acids of the carboxy ends of the α, β and γ chains of normal fibrin(ogen), as well as that of the recently discovered αE chain, the so-called extended αE segment (αE) (Fu, Y. and Grieninger, G. "Fib420 : A normal human variant of fibrinogen with two extended α chains", Proc.Natl.Acad.Sci. USA, 91: 2625-2628; (1994)). Sequences of such peptides are given in Table 1 below. Sequence 09 is in accordance with the present invention; the others are comparisons.

**Table 1. Synthetic peptides corresponding to the carboxy termini of fibrinogen.**

| Code # | Chain Address | Sequence |
|---|---|---|
| 07 | α 590-610 | EADHEGTHSTKRGHAKSRP |
| 09 | β 441-461 | KGSWYSMRKMSMKIRPFFPQQ |
| 70 | γ 391-411 | LTIGEGQQHHLGGAKQAGDV |
| 71 | αE 827-847 | RGADYSLRAVRMKIRPLVTQ |

Hereinafter, the term "peptide" refers to peptides-09, 70 or 71, having a sequence selected from the group consisting of:
KGSWYSMRKMSMKIRPFFPQQ, LTIGEGQQHHLGGAKQAGDV or RGADYSLRAVRMKIRPLVTQ; as well as to analogues, equivalents or peptido-mimetics thereof, or fragments thereof displaying substantially identical or similar functional activity as one of the above-listed sequences.

The term "peptides of the present invention" refers to peptides as defined) in and by the appended claims as well as comparison peptides. The mitogenic effects of these peptides were tested in cell culture systems. No effects were observed with peptide-07. Also, only peptide-71 had cell proliferative effects. These peptides were also evaluated for their adhesive properties when bound to sepharose beads (SB) placed on nearly confluent cell cultures. Specifically, peptide-09 had the greatest adhesive capability. The next most potent peptide/bead combination featured peptide-71. Peptide-70 appeared to be haptotactic for EC. Thus, when tested in cell culture systems, with the exception of peptide-07, the peptides were clearly shown to have cell adhesive effects and one peptide also showed some cell proliferative effects.

The two most potent peptides, peptides 09 and 71, share an underlined sequence of YSXRXXMKIRPXXXQ. The shared sequence itself, possibly with the addition of a spacer moiety or moieties for proper geometrical configuration, is also contemplated as a peptide of the present invention.

The peptides of the present invention are contemplated for many different uses. Therapeutic uses include, but are not limited to, treatment of a wound bed. Methods for treatment of the wound bed with the peptides of the present invention are given in greater detail in Example 3 below. In addition, therapeutic compositions which include the peptides of the present invention are given in greater detail in Example 4 below.

Additional uses of the peptides of the present invention include, but are not limited to, the growth and transport of cells in cell culturing systems, the separation of different types of cells from mixed cell cultures, and the transplantation of cells into tissues or cell cultures. These uses are explained in greater detail in Example 2 below. Furthermore, as explained in greater detail in Example 5. below, the peptides of the present invention can also be used as tools for biological analysis and for further research and development.

These contemplated uses of the peptides of the present invention are intended as illustrations only and are not meant to be limiting in any way.

In addition to these uses for the peptides, the equivalent nucleotide sequences are also contemplated as having utility. The term "equivalent nucleotide sequence" refers to the sequence of a polynucleotide which could encode for at least one of the amino acid sequences of the peptides of the present invention. Such a polynucleotide could be a DNA (deoxyribonucleic acid) or RNA (ribonucleic acid) sequence, for example. The equivalent nucleotide sequence could be used in a diagnostic kit, for example to diagnose the inability of a subject to develop and dissolve clots appropriately. Thus, the equivalent nucleotide sequences of the present invention also have utility within the scope of the present invention.

### DETAILED DESCRIPTION OF THE PRESENT INVENTION

The present invention is drawn towards novel peptidic sequences of fibrin, and related nucleotide sequences thereof. Methods of using these peptidic and nucleotide sequences are also contemplated, including methods for the promotion of wound healing as well as diagnostic methods. These peptidic sequences retain desirable properties exhibited by the entire fibrin molecule, such as cell adhesion.

The principles and operation of such peptidic amino acid sequences of fibrin and related nucleotide sequences according to the present invention may be better understood with reference to the non-limiting illustrative examples below.

### Example 1

### Synthesis and Analysis of the

### Effects of the Peptidic Fragments

The peptides of the present invention were synthesized and tested in cell culture systems as described below. The experimental procedure is described in the section entitled "Experimental Procedure". The results are given in the section entitled "Results".

Essentially, peptides designated peptide-07, peptide-09, peptide-70 and peptide-71 were synthesized and covalently attached to sepharose beads, to form SB-07, SB-09, SB-70 and SB-71, respectively. Fibrinogen was also covalently attached to sepharose beads, to form SB-Fib. The bead-peptide combination was then incubated with cultured cells. The data are shown in the "Results" section below. In summary, SB-09 appeared to be the most potent for binding, showing even greater potency than SB-Fib for all cell lines which bind fibrin(ogen). All tested cell lines bound to SB-09 under these conditions, with the exception of OV-1063, keratinocytes and cells derived from a leucocytic lineage. The next most potent peptide/bead combination was SB-71, as strong binding was observed for SB-71 for the following cell lines: HF, MF, EC and EMT-6. Weak binding of SMC cells to SB-71 was also observed. SB-70 appeared to be specific for EC, although variable binding was observed, presumably due to degradation of a specific receptor during passage and handling of these cells. No cell binding was observed to SB-07.

Interestingly, of the four peptides tested only peptide-71 appeared to induce cell proliferation. Furthermore, FITC-labeled peptides-71 and 09 were clearly able to bind to the cell membrane, and after a prolonged exposure, accumulated in the cytoplasm, and migrated to the perinuclear area and granular bodies.

### Experimental Procedure

### Preparation of Peptides

Peptidic analogues of the carboxy termini fibrinogen were synthesized according to standard techniques (Synthetic Peptide Corp., Dublin, California, USA). The amino acid sequences of these analogues are given in Table 1 above. Each peptide was labeled with a fluorescent label which was either fluorescein or EACA (epsilon amino caproic acid). The labeling was performed according to well known methods in the art, for example during the synthesis of the peptide itself.

### Preparation of sepharose Beads With Bound Peptides or Proteins.

Peptides or fibrinogen were covalently bound to CNBr activated sepharose beads (SB) (Pharmacia, Piscataway, NJ) using techniques previously used to bind albumin, fibrinogen and thrombin. Briefly, CNBr-activated sepharose 4B (Pharmacia) was washed with IN HCI, and suspended in the coupling buffer (pH 8.3), following the protocol supplied by the manufacturer. The peptides were dialyzed against the coupling buffer to remove Tris. Peptides (1 mg) were not highly water soluble. The peptides were dissolved in 1 mL 70% ethanol or dimethylformamide, and were then mixed into the coupling buffer containing 1 mL CNBr-activated sepharose. The suspension was gently agitated overnight, then centrifuged 500 x g to pack the beads. This procedure covalently binds more than 95% fibrinogen or peptides-07 and 09 to the beads determined by Abs₂₈₀ readings of the solutions. Concentrations of peptides bound to SB were: peptide 07 was 7 mg/mL; peptide 09 was 5.2 mg/mL; peptide 70 was 9.6 mg/mL; peptide 71 was 6.0 mg/mL. The beads were washed with Tris-saline buffer and stored at 4°C with 0.1 % azide. Before use, the beads were washed 3 times in sterile saline to remove all traces of azide.

After incubation with cells, samples of beads were prepared for scanning electron microscopy (SEM) by fixing with 4% glutaraldehyde, critical point dried, and coated with osmium tetroxide, sputter coated with Au/Pd and then examined with a Hitachi S-530 Scanning Microscope.

### Preparation of Cell cultures.

All cell cultures were prepared as previously described. Normal human skin fibroblasts (HF) were isolated from skin biopsies of young human subjects. The dermal layer of skin was chopped and digested 1 hour by 0.25% trypsin/versen. The isolated cells were washed and plated on plastic Petri dishes with DMEM supplemented by 10 % fetal calf serum (FCS), antibiotics, and glutamine. The plates were washed after 10 hrs to select for the better attached fibroblasts. At the 3-4 passage, the cells consisted of homogeneous populations of fibroblasts, as judged by microscopy. Immunohistology with monoclonal antihuman-fibroblast-surface proteins confirm that this procedure yields homogeneous fibroblast culture (Ronnov-Jessen L, Celis JE, Van-Deurs B, Petersen OW: "A fibroblast-associated antigen: characterization in fibroblasts and immunoreactivity in smooth muscle differentiated stromal cells", J. Histochem. Cytochem. 40: 475-486 (1992)). Normal murine fibroblasts (MF) were isolated from the skin of 2-3 days old neonate C3H mice by 3 step digestion, each for 2 hrs, with trypsin/versen. The use of neonate mice with low cross linking of collagen served to enhance the high yield of cells during the proteolytic digestion. The details of the rest of the protocol are similar to those used for the isolation and growth of HF. These cells could be grown for at least 12-14 passages before any decrease of the rate of proliferation occurred. Cells were used from the fourth passage to the tenth passage.

Porcine smooth muscle cells (SMC) were isolated from thoracic aortas of young animals and kept in culture with twice weekly medium change and splitting once in 1-2 week. Cells of up to 10 passages were used. The purity of the SMC culture was verified by immunohistology with monoclonal anti muscle-specific-actin HHF-35 (Bar-Shavit R, Benezra M, Eldor A, Hy-Am E, Fenton JW, Wilner GD & Vlodavsky I: "Thrombin immobilized to extracellular matrix is a potent mitogen for vascular smooth muscle cells: nonenzymatic mode of action", Cell Regul. 1: 453-463, 1990). Other cell lines were obtained from different sources and cultured in their standard conditions as described in the following references: murine fibroblast line (3T3) and normal human keratinocytes (Ben-Bassat H, Eldad A, Chaouat M, Livoff A, Ron N, Neeman Z, and Wexler MR: "Structural and functional evaluation of modifications in the composite skin graft: cryopreserved dermis and cultured keratinocytes", Plastic & reconstructive Surgery 89: 510-520 (1992)); murine mast cells (MC-9) (Razin E, and Marx G., "Thrombin-induced degranulation of cultured bone marrow-derived mast cells", J. Immunol. 133: 3282-3285 (1984)); normal bovine aortic endothelial cells (BAEC) (Vlodavsky I, Greenburg G, Johnson LK and Gospodarowicz D: "Vascular endothelial cells maintained in the absence of fibroblast growth factor undergo structural and functional alterations that are incompatible with their in vivo differentiated properties", J. Cell Biol. 83:468-486, 1979); porcine smooth muscle cells, isolated and cultured as previously described (Bar-Shavit R, Benezra M, Eldor A, Hy-Am E, Fenton JW, Wilner GD & Vlodavsky I: "Thrombin immobilized to extracellular matrix is a potent mitogen for vascular smooth muscle cells: nonenzymatic mode of action", Cell Regul. 1: 453-463, 1990); murine leukemic cells (P-388) (Ramu A, Ramu N.& Gorodestsky R, "Reduced oubain resistant potassium entry as a possible mechanism of multidrug-resistance in p388 cells", Biochem. Parmacol., 42: 1699-1704 (1992)); human ovarian carcinoma cells (OV-1063) were isolated from a primary tumor and then maintained as previously described (Horowitz AT, Treves AJ, Voss R, Okon E, Fuks Z, Davidson L, and Biran S., "A new human carcinoma cell line: establishment and analysis of tumor associated markers", Oncology 42: 332-337 (1985)); murine mammary adenocarcinoma cells (EMT-6) were grown under the standard conditions (Rockwell S., "Cytotoxic and radiosensitizing effects of hypoxic cell sensitizers on EMT6 mouse mammary tumor cells in vivo and in vitro", Br J Cancer 37: 212-215 (1978)); and the murine macrophage-like cells (J774.2) (Ringel R, Roy SN, and Horwitz SB., "A phosphoglycoprotein associated with taxol resistance in J774.2 cells", Cancer Res. 45: 3856-3863 (1985)).

All culture medium components were purchased from Biological Industries (Beit-HaEmek, Israel) and fetal calf serum was supplied by GIBCO (Grand Island, New York, NY, USA). The cell cultures were maintained at 37°C in a water-jacketed CO₂ incubators, and were harvested by trypsin/versen solution with 1-2 passages per week in a split ratio of 1:10 for fast proliferating transformed cells and 1:4 for normal cell types.

### Assays for Cell proliferation

Cell proliferation was evaluated by measuring cell density as a function of time by two different colorimetric assays. The MTS colorimetric assay (CellTitre 96 Aqueous Assay by Promega) is based on dehydrogenase conversion of MTS (methyl tetrazolium salt) by viable cells to colored tetrazolium salt (Ge M., Tang, G., Ryan, T.J. and Malik, A.B., "Fibrinogen degradation product fragment D induces endothelial cell detachment by activation of cell-mediated fibrinolysis", J.Clin.Inves., 90:2508-2516 (1992)); the methylene blue (MB) assay is based on the staining of monolayer cells after their fixation, and reading the absorbence of the absorbed dye.

The MTS assay for viable cells was performed as follows: 30 µL of freshly prepared MTS/PMS were added to each well; following 2 hrs incubation at 37 °C, the plates were placed on a computer driven microplate reader (Anthos HT-II, Salzburg, Austria), programmed to shake the plate for 1 minute before reading the optical density (OD) of the dye at 490 nm. The measurements were repeated following 4 and 6 hours of incubation.

For the MB assay, cells were fixed at the end of the experiment by the addition of 50 µL 2.5% gluteraldehyde for 10-15 min. The plate was then washed 10 times in deionized water, once with borate buffer (0.1 M, pH 8.5), dried and stained with MB (100 µL of 1% solution in borate buffer), incubated for 2 hour at ambient temperature. Cells were rinsed exhaustively to remove unbound dye and air dried. The MB dye bound to the cells in each well was extracted by incubating with 200 µL of 0.1 N HCl for 1 hr at 37 °C, and read at 620 nm. For each experiment, 3 wells were used for each condition tested. The net absorption by the methylene blue assay was shown previously to correlate (r>0.99) with the cell density on the plate. Every point of each experiment was the average reading of 3 wells; the data points in the graphs represent 2-3 repetitive experiments of both assays.

Typically for both assays, the effect of added peptides relative to controls were similar at 72 and 96 hours and could be averaged.

### Assay of cell adhesion to peptides bound to sepharose beads (SB).

The migratory/adhesion response of cells to proteins (such as fibrinogen) or peptides covalently bound to SB was measured as follows. Cells were grown in 6 or 12 well plates to near confluence or in suspension until they covered about 1/2 to 2/3 of the plate surface. At that point, about 20-150 mL of a suspension containing 50% v/v SB coated with the test protein or peptide were added to the plate and dispersed by gentle shaking for 1 min. The plate was then returned to the incubator and examined at different times by inverted phase microscopy.

The SB (coated or uncoated) sedimented into the nearly confluent cell layer and occasionally made physical contact with cell membranes on the plate (ascribed to Brownian motion or micro-convection currents). In a positively responding system, this resulted in the tethering of SB to the cell layer, which could be detected by visual inspection at different time points. Typically, 300 beads (but not less then 200) were counted in each well and the ratio of SB bound to the cells relative to total number SB could be calculated. Counting the percentage of SB attached to the cell surface at different time intervals provided a quantitative assay of the kinetics of the attachment of coated beads to cells. Negative control with uncoated SB or positive controls with SB-fibrinogen were employed with at least 3 wells measured for each. The statistical error was calculated from the square root of the total counts.

### Electron microscopy

Samples of the cells with attached sepharose beads were fixed as described previously, and were then examined with a Hitachi S-530 Scanning Microscope (SEM).

### Fluorescence Microscopy

The cells examined were grown in 6-well plates on cover slips to reach near confluence. At the time of examination, the cover slips were inverted and put on a microscope slide supported by 2 thin spacers so that a thin gap (~2 mm) was left between the cells on the coverslip and the slide. This was filled with culture medium. To follow the uptake, 10 µg/mL FITC-labeled peptide (09) was added into the culture medium in the gap. At different time points, medium was replaced with fresh medium and the fluorescence was viewed and photographed, using an Olympus fluorescent microscope system.

### Results

### Attachment of Fibrinogen- or Peptide Coated sepharose Beads to Cells

SB-Fibrinogen or peptide coated beads were incubated with near confluent cells. Control "naked" SB did not interact with the cell layers and floated freely in the culture medium even after prolonged incubation, resulting in zero percent attachment to the cells. The percentage of SB-Fib/peptide attached to cells (by day 4) is given in columns 2-6 of Table 2.

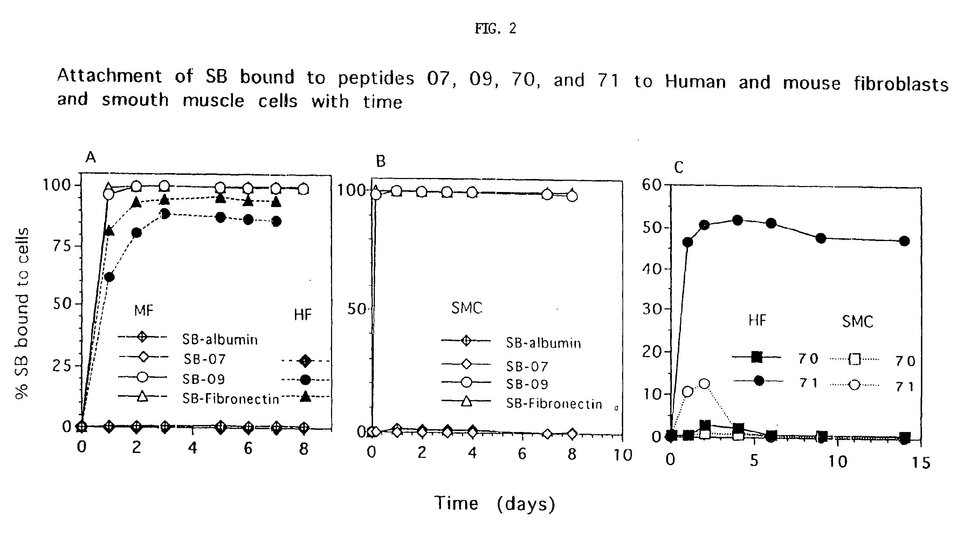

Sepharose beads coated with fibrinogen or one of the synthetic peptide analogs of the four carboxy termini of fibrinogen were active in attracting and binding to the cells of most cell lines tested, as shown in Table 2 and Figure 2. For example, sepharose beads coated with peptide 09 (SB-09) bound to 5 out of 6 cell lines and with higher efficacy than SB-Fib. Sepharose beads coated with peptide 71 (SB-71) bound to 4 out of 6 cell lines. Sepharose beads coated with peptide 70 (SB-70) bound to 1 out of 6 cell lines. Sepharose beads coated with peptide 07 (SB-07) bound to none. In some cases, strong binding to endothelial cells (EC) was observed, but this response was not reproducible.

**Table 2. % Binding of peptide-coated SB to normal cultured cells.**

| **(Day 4)** | | | | | |
|---|---|---|---|---|---|
| | | **Peptides** | | | |
| **Cell Line** | **SB-Fib** | **SB-07** | **SB-09** | **SB-70** | **SB-71** |
| **HF** | 71 | 0 | 100 | 2 | 52 |
| **MF** | 81 1 | 0 | 89 | 0 | 61 |
| **SMC** | 74 | 0 | 100 | 1 | 8 |
| **EC** | 70 | 0 | 94 | 2 to 93* | 43 |
| **EMT-6** | | 1 | 99 | 2 | 64 |
| **OV-1063** | | 0 | 4 | 0 | 0 |

| | | | | | |
|---|---|---|---|---|---|
| *Response varied with different cell batches. | | | | | |

Figure 2 shows the haptotactic effect (attachment) of the peptides of the present invention for various cell types. Figure 2A shows the effect of SB-07, SB-09, SB-fibronectin or SB-albumin on mouse and human fibroblasts. Figure 2B shows the effect of SB-07, SB-09, SB-fibronectin or SB-albumin on smooth muscle cells. Figure 2C shows the effect of SB-70 or SB-71 on smooth muscle cells and human fibroblasts. Fibronectin is known to be haptotactic, while albumin is not. SB-albumin and SB-07 do not bind to any of the cell types. SB-09 and SB-fibronectin bound to all cell types tested. SB-71 bound to human fibroblasts, although with somewhat less activity than SB-09, but only transiently bound to smooth muscle cells. SB-70 had only minor binding to human fibroblasts and smooth muscle cells.

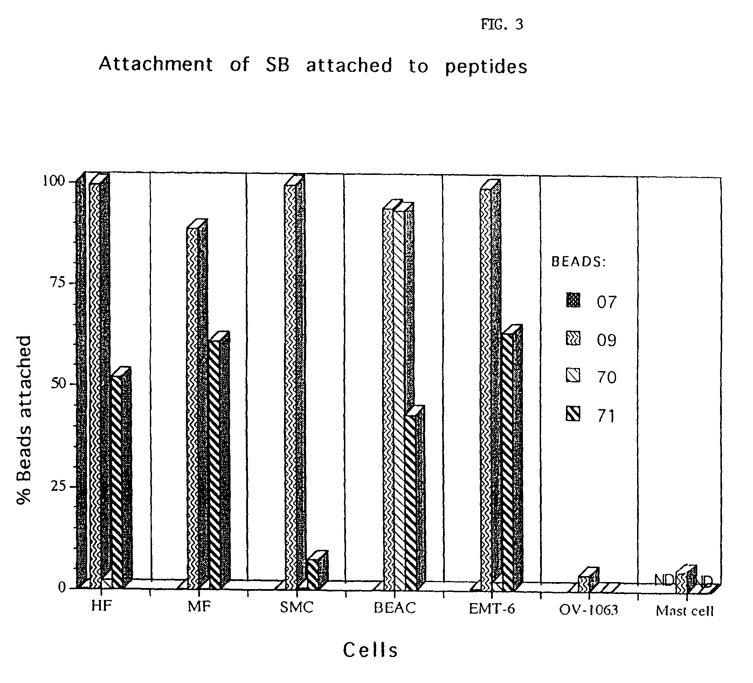

Figure 3 shows the percentage attachment of various cell types to SB-peptides of the present invention. SB-07 again did not bind to any of the cell types. SB-09 bound to all tested cell types, except for the OV-1063 cell line and mast cells. SB-70 bound only to endothelial cells. SB-71 bound to all cell types which bound to SB-09, but to a lesser extent.

### Effect of the peptides on cell proliferation

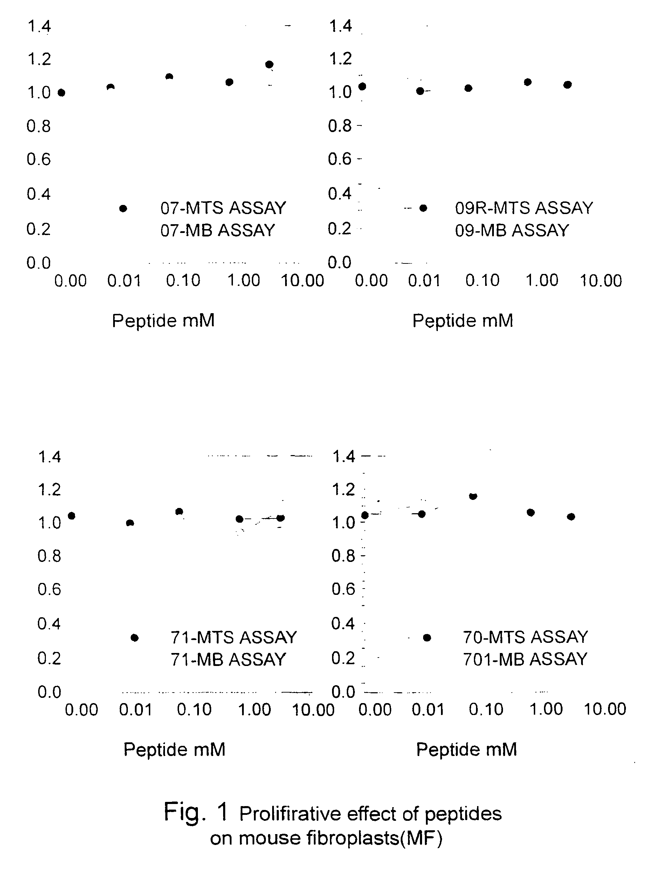

Peptides up to 1 µg/mL or 100 µg/mL fibrinogen or thrombin (final concentrations) were added to the culture medium and cell numbers were assayed by day 3 using the MTS assay. The change in cell number was compared with that observed versus saline control. The proliferative effect of the tested peptides, fibrinogen and thrombin was determined with the MTS colorimetric assay. Of the 4 peptides tested at dosages up to 1 µg/mL (approximately equimolar to 100 µg/mL fibrinogen), peptide-71 was able to promote cell proliferation of HF and MF, while the remaining peptides exerted little effect on cell proliferation (Table 3 and Figure 1).

**Table 3. Proliferative effects of fibrinogen and peptides on cultured cells.**

| Cell | | | | |
|---|---|---|---|---|
| Compound | Chains | HF | MF | SMC |
| Thrombin | NA | + | + | + |
| Fibrinogen | α, β, γ | - | -+ | + |
| 7 | α | - | - | - |
| 9 | β | - | - | - |
| 70 | γ | - | - | - |
| 71 | αE | - | - | +- |

### Uptake of FITC-09 and FITC-71 by cells

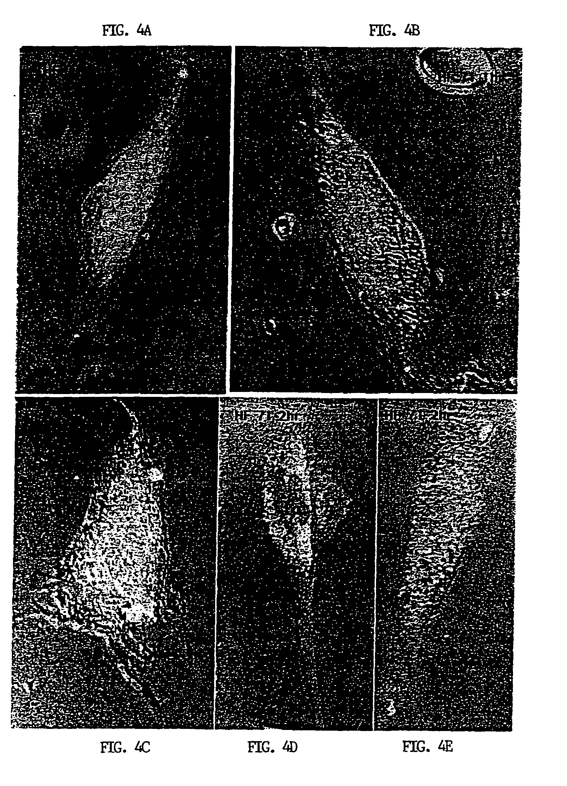

Exposure of cultured human fibroblast cells to a solution of 10 micromolar peptide FITC-71 resulted in uptake by human fibroblasts, at one (Figures 4A and 4B) and two hours of incubation (Figures 4C-E) as shown by fluoro-microscopy. Accumulation of the FITC-peptide in the cytoplasm and around the nucleus was clearly observed

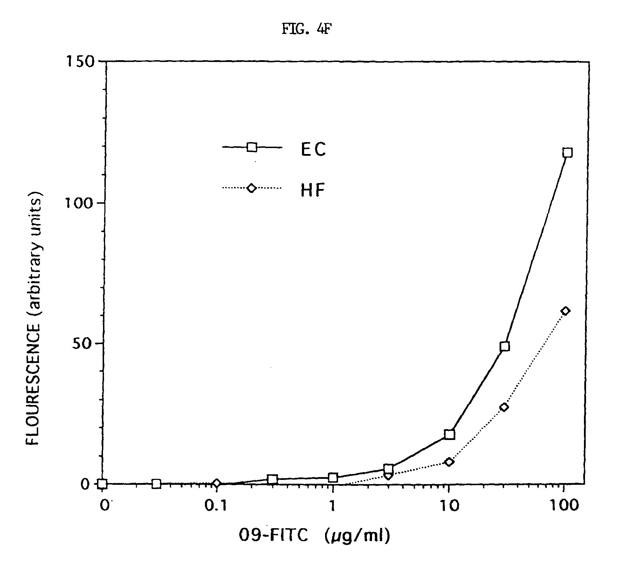

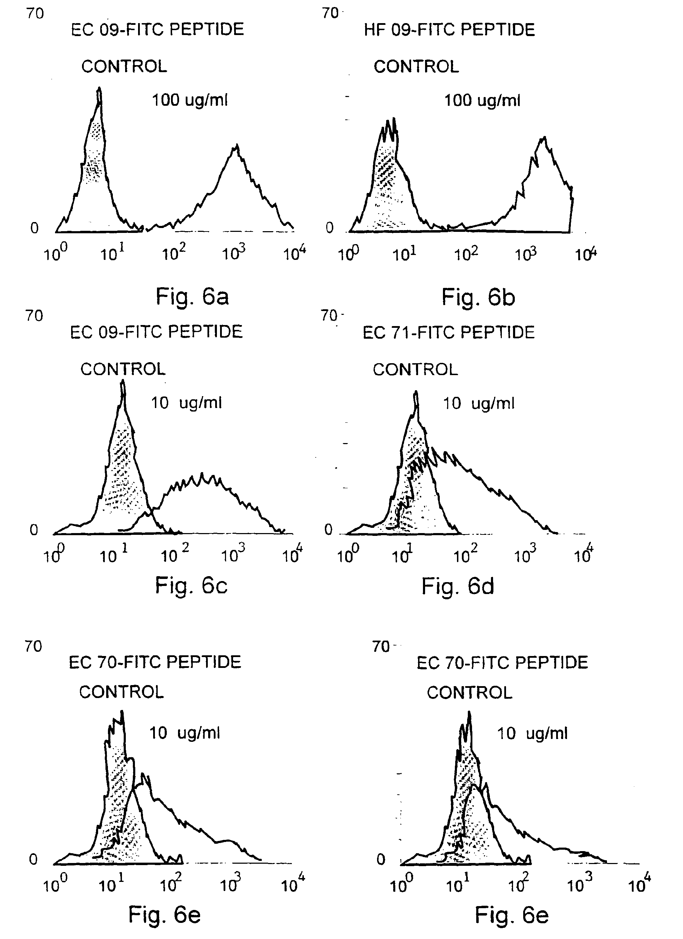

Exposure of cultured HF, EC and SMC cells to solutions of 10 micromolar peptide FITC-09 resulted in significant uptake similar to that seen with peptide-71. After a short exposure of the cells to 5-10 micromolar of peptide FITC-09, the FITC-peptide was observed to bind to the cell membrane. After a longer exposure of more than 1 hour or with fixed cells, accumulation of the FITC-peptide in the cytoplasm and around the nucleus was clearly observed (data not shown). In most cases, the fluorescence became concentrated in discrete cytoplasmic vesicles. The uptake was shown to be concentration dependent for EC cells (Figure 4F).

### FACS Analysis of FITC-labeled peptides-09, 71, 70 and 07

EC and HF cell monolayers were washed and then incubated with trypsin-versene. Cells were then washed with growth medium and resuspended in medium with 0.1% albumin. FITC-labeled peptide-09 was incubated with 5x10⁵ EC cells (10 micrograms/ml or 100 micrograms/ml) or with 2.5x10⁵ HF cells (100 micrograms/ml) in medium with 0.1% albumin. FITC-labeled peptide-71, 07 and 70 (10 micrograms/ml) was incubated with 5x10⁵ EC cells in medium with 0.1 % albumin. Cells were then washed with PBS and 1% albumin. Cells were resuspended in PBS and 1% albumin and then filtered through a mesh for FACS analysis, in which the FITC fluorescence was measured for each cell.

Figure 5 shows FACS analysis of FITC-labeled peptides-09 (Figures 5A-5C), 70 (Figure 5E), 71 (Figure 5D) and 07 (Figure 5F). The x-axis shows fluorescence in arbitrary units and the y-axis shows number of cells. The control is without FITC-labeled peptide (background fluorescence). Similar binding was seen to EC and HF cells with FITC-labeled peptide-09. A lower concentration of FITC-labeled peptide-09 bound with greatest activity to EC, followed by FITC-labeled peptide-71. FITC-labeled peptide-70, showed slight binding to, and 07 bound non-specifically to, EC cells.

### Example 2

### Structured Cell Systems Using the Peptides of the Present Invention

The peptides of the present invention could be used as part of structured cell systems, for example as for tissue engineering. The cell system of the present invention includes at least one type of cell bound to at least one peptide of the present invention. Suitable types of cells include any cells which are capable of binding to at least one peptide of the present invention. Examples of such cells may include, but are not limited to, fibroblasts, endothelial cells, chondrocytes, neuroblastoma cells, melanoma cells, kidney cells, liver cells, pancreatic cells, thyroid cells, glial cells, smooth muscle cells, mouse mammary carcinoma cells, bone or cartilage forming cells, and combinations thereof.

The cell system of the present invention could also be used to culture cells as part of a cell culture system. At least one type of cell would be allowed to bind to the peptide of the present invention. The cells would then be grown in a culture medium under suitable conditions for cell culture. The advantage of such a cell culture system is that the peptide of the present invention could be attached to a suitable structure, such as a sepharose bead or any other geometrically suitable structure. Hereinafter, the term "structure" includes but is not limited to the term "matrix".

The cells would then grow on that structure, rather than on a conventional Petri dish. Therefore, when the cells are to be removed from the culture medium, the structure itself would be removed substantially without trauma to the cells. By contrast, conventional methods for removing cells from culture medium often require trypsinization, which may damage certain receptors on the cells and otherwise cause trauma to the cells. The ability to transfer cells from one environment to another by moving such a structure or matrix also enables the cells to be re-seeded into fresh culture medium with minimal damage to the cells.

Such a cell culture system which incorporates the peptides of the present invention could also be used to culture the cells at a higher density than conventional cell cultures. Such high density cell cultures are particularly useful for the production of recombinant proteins and for other types of cell culture products. The cell culture system of the present invention could be used to culture cells transformed or transfected with various vectors, viruses, nucleic acids and the like, in order to facilitate the production of these cell culture products.

The peptides of the present invention could also be used to separate cells which are capable of binding to one or more of these peptides from those cells which are not capable of binding to such peptide(s). For example, a peptide of the present invention could be attached to a matrix or structure as described previously. This structure or matrix could then be incubated with a mixture of cells in solution, under suitable conditions to enable binding of those cells which are capable of binding to the peptide. The structure or matrix could then be removed, and with it the bound cells. Cells which are not capable of binding to the peptide, whether because these cells are of a type which does not bind or because the cells have been damaged in some way, would remain behind. As an additional example, a peptide of the present invention could be attached to a substantially immobile support (such as the surface of a prosthetic device), and the solution of cells could be placed in contact with it. Those cells which are capable of binding to the peptide would remain bound to the support, while the other cells would be removed. Thus, the peptides of the present invention could be used for separation of cells and for coating the surface of such a device.

Such separation may also prove useful for diagnostic purposes. For example, the presence or absence of a certain type of cell, or of a certain cell function, could be determined by examining whether the cell bound to the peptide. The presence or absence of substantially any cell type which binds to one or more of the peptides of the present invention could be determined in such an assay (such as by fluorescent cell sorter analysis). Examples of cell functions which be diagnosed include, but are not limited to, the ability to respond to a chemotactic or an attachment signal.

These functions would be assessed by the determination of cell binding to the peptide-coated structure. The determination of cell binding could be performed by the ability to separate the cell from a solution or mixture of different cell types, as described previously.

Alternatively and preferably, the peptide could be labeled with a reporter, such as a fluorescent or radioactive moiety. The reporter would be used to determine if the peptide had bound to any of the cells, thus enabling the presence or absence of the cell type, or of a certain cell function, to be determined.

Examples of suitable fluorescent moieties include, but are not limited to, FITC (fluorescein), rhodamine and Texas red. Examples of suitable radioactive moieties include, but are not limited to, phosphorous 32, iodine 131 and tritium. The reporter could be attached to the peptide during synthesis or alternatively post-synthesis, according to well known methods in the art.

The peptides of the present invention are also contemplated as being useful for the formation of a therapeutic structure of cells. Examples of the therapeutic structure include, but are not limited to, a gel, a prosthetic device, and a collagen sheet. At least one peptide of the present invention would be attached to the therapeutic structure, for example by a covalent bond formed with a chemical cross-linking reagent as is well known in the art, or with fibrin glue. The cells would then be allowed to bind to the peptide on the therapeutic structure, for example through cell culture or by the separation methods described above. The choice of cells will depend upon the type of tissue being contacted and the desired therapeutic structure, and could potentially include any cell type which is capable of binding to at least one peptide of the present invention.

### Example 3

### Methods for Treatment with

### the Peptides of the Present Invention

The peptides of the present invention are contemplated as being useful for treatment of a subject with a disease condition, in which the condition can be ameliorated or cured, at least in part, through cell chemotaxis or proliferation, or by transplantation of cells. Examples of such a condition include, but are not limited to, the presence of a wound and diseases characterized by an absence of a cell product. The term "wound" includes any disruption of the normal integrity of an organ of the subject. Examples of such an organ include, but are not limited to, the skin, the abdominal cavity, the intestine, the heart, the lungs, any blood vessel, any bone, the pancreas, the liver, a kidney, the reproductive organs or the stomach.

The wound may be present as the result of a surgical intervention or as the result of a non-surgical intervention. The surgical intervention could be either planned or as the result of a medical emergency. The non-surgical intervention could be a burn, an ulcer, a laceration or any type of accidental injury or trauma.

Methods of treatment with the peptides of the present invention for surgical intervention could include placing one or more of the peptides at the site of the surgical intervention, in order to increase the efficiency of the wound healing process. The one or more peptides could be placed at the site of the surgical intervention before surgery, particularly for emergency surgery, during surgery or after surgery. The one or more peptides could be included in a therapeutic composition, as described in Example 4 below.

Methods of treatment of non-surgical interventions would include placing one or more of the peptides at the site of the non-surgical intervention, in order to increase the efficiency of the wound healing process. The one or more peptides could also be included in a therapeutic composition, as described in Example 4 below.

The one or more peptides of the present invention could be placed at the site of the surgical or non-surgical intervention once, or repeatedly, depending upon the type and gravity of the wound which was sustained. The concentration and rate of treatment, if repeated, could easily be determined by one of ordinary skill in the art.

Examples of diseases characterized by an absence of a cell product include, but are not limited to, diabetes mellitus, hemophilia A (factor VIII deficiency), and hemophilia B (factor IX) deficiency. These diseases could be ameliorated or cured by introducing cells which produce the necessary cell product or products into the subject. These cells could be prepared by introduction of a vector containing the nucleic acid sequence coding for a protein or peptide, for example, as is well known in the art. The peptide or protein could itself be the desired cell product, such as insulin. Alternatively and preferably, the protein could cause the cell to produce the desired cell product, for example through an enzymatic reaction or reactions. In any case, the cell would then be able to produce the desired cell product after such preparation.

Once prepared, the cells would be bound to a peptide of the present invention, which would in turn be incorporated within a suitable cell structure as described in Example 2. The cell structure would be administered to the subject and would then produce the necessary cell product. The advantage of such a cell structure according to the present invention is that the cells would remain substantially localized, although the cell products could be enabled to enter the bloodstream if desired. Thus, by using the peptide of the present invention, the cell structure could be used to treat the disease condition with the necessary cell product or products.

### Example 4

### Suitable Formulations for Administration of the Peptides

The peptides of the present invention can be administered to a subject in a number of ways, which are well known in the art. Hereinafter, the term "subject" refers to the human or lower animal to whom the peptide was administered. For example, administration may be done topically (including opthalmically, vaginally, rectally, intranasally), orally, or parenterally, for example by intravenous drip or intraperitoneal, subcutaneous, or intramuscular injection.

Formulations for topical administration may include but are not limited to lotions, ointments, gels, creams, suppositories, drops, liquids, sprays and powders. Conventional pharmaceutical carriers, aqueous, powder or oily bases, thickeners and the like may be necessary or desirable.

Compositions for oral administration include powders or granules, suspensions or solutions in water or non-aqueous media, sachets, capsules or tablets. Thickeners, diluents, flavorings, dispersing aids, emulsifiers or binders may be desirable.

Formulations for parenteral administration may include but are not limited to sterile aqueous solutions which may also contain buffers, diluents and other suitable additives.

The peptides of the present invention may be placed on the wound bed as part of a composition for wound treatment. The composition for wound treatment can include a suitable pharmaceutically acceptable carrier. For example, the peptide could be incorporated into lasered or heated albumin to accelerate wound healing, minimize scarring, accelerate the rate of deposition of new extracellular matrix and augment angiogenesis.

As another example, a polymer could be made of subunits of at least one of the peptides of the present invention, such that a plurality of these peptides would be linked to form the peptide polymer. The peptides could be linked with a chemical cross-linking moiety, for example. More than one of the peptides of the present invention could be used to form the polymer. Alternatively, at least one of the peptides of the present invention could be attached to a biologically acceptable synthetic polymer, again through a suitable cross-linking moiety, to form a co-polymer. In either case, the resultant peptide polymer or co-polymer could be used to fabricate microparticles which could either be included in a composition according to the present invention, or else could be used form cell structures as described in Example 2 above.

The composition for wound treatment can also include at least one bioactive agent. Suitable bioactive agents include, but are not limited to, drugs, neurologics, vitamins, vitamin derivatives, growth factors, glucocorticosteroids, steroids, antibiotics, antibacterial compounds including bacteriocidal and bacteriostatic compounds, antiparasitic compounds, tumoricidal compounds, tumoristatic compounds, toxins, enzymes, enzyme inhibitors, proteins, peptides, minerals, neurotransmitters, lipoproteins, glycoproteins, immunomodulators, immunoglobulins and fragments thereof, fatty acid derivatives, polysaccharides, cell receptor binding molecules, anti-inflammatories, anti-glaucomic compounds, mydriatic compounds, anesthetics, nucleic acids, polynucleotides and the like.

The therapeutic composition could also include at least one type of cell in a structured format, as described in Example 2 above. For example, the previously described sheet structure for cell culture could be placed on the wound in order to both protect the wound during the healing process, and to promote the wound healing process itself. The structure could also be the previously described gel, which would be placed on the wound for transplanting the cells onto the site of the wound, and would then be able to promote the wound healing process. Other examples of such structured cell systems could also be used as part of the therapeutic composition of the present invention for wound healing. When used for wound healing, suitable cell types include, but are not limited to, fibroblasts, smooth muscle cells, endothelial cells, chondrocytes, bone or cartilage forming cells, and combinations thereof.

Combinations of any two or more of these different components of therapeutic compositions are also possible as therapeutic compositions of the present invention.

Dosing is dependent on the severity of the symptoms and on the responsiveness of the subject to the peptide or fragments of the present invention. Persons of ordinary skill in the art can easily determine optimum dosages, dosing methodologies and repetition rates.

### Example 5

### Analysis Performed with the Peptides of the Present Invention

The peptides of the present invention are also contemplated as tools for performing analysis of other systems, and for further research and development. For example, the peptides could be used to identify and isolate cell receptors. As described previously, the peptide could be labeled with a reporter, such as a fluorescent or radioactive moiety. The reporter would be used to determine if the peptide had bound to any of the cells, thus enabling the presence or absence of the cell type, or of a certain cell function, to be determined.

Examples of suitable fluorescent moieties include, but are not limited to, FITC (fluorescein), rhodamine and Texas red. Examples of suitable radioactive moieties include, but are not limited to, phosphorous 32, iodine 131 and tritium. The reporter could be attached to the peptide during synthesis or alternatively post-synthesis, according to well known methods in the art. Thus, the ability of the peptide to bind to a novel receptor or other protein could be determined according to a binding assay.

In addition, the peptides of the present invention could be used to design analogues, such as non-peptide mimetics, of these peptides. Such non-peptide mimetics could be used for therapeutic purposes, for example. Non-peptide compounds are potentially easier to administer, since peptides are preferably administered nasally or parenterally, for example, while non-peptide compounds could potentially be administered orally. Furthermore, particular properties of each peptide could be selected or augmented by designing a specific analogue. Thus, the peptides of the present invention could potentially yield many new and different types of therapeutic medicaments.

## Claims

1. A peptide derived from fibrinogen, consisting of a chain of amino acids having a sequence of KGSWYSMRKMSMKIRPFFPQQ, or a haptotactic fragment thereof.

2. A composition, comprising a peptide derived from fibrinogen consisting of a chain of amino acids having a sequence of KGSWYSMRKMSMKIRPFFPQQ, or a haptotactic fragment thereof, further comprising a pharmaceutically acceptable carrier.

3. The composition of claim 2, further comprising a biological agent selected from the group consisting of drugs, vitamins, vitamin derivatives, growth factors, glucocorticosteroids, steroids, antibiotics, toxins, enzymes, enzyme inhibitors, immunomodulators, immunoglobulins and fragments thereof, fatty acid derivatives, polysaccharides, cell receptor binding molecules, anti-inflammatory agents, nucleic acids, and polynucleotides.

4. The composition of claim 2, further comprising a cell selected from the group consisting of fibroblasts, endothelial cells, chondrocytes, neuroblastoma cells, kidney cells, liver cells, pancreatic cells, thyroid cells, glial cells, smooth muscle cells, mouse mammary carcinoma cells, bone or cartilage forming cells, and combinations thereof.

5. The composition of claim 4, wherein the cell type is selected from the group consisting of fibroblasts, smooth muscle cells, endothelial cells, chondrocytes, and combinations thereof.

6. The composition of claim 5, wherein the cell type is a fibroblast.

7. Use of a composition as claimed in any one of claims 2 to 6 for the manufacture of a medicament for promoting healing of a wound in a subject.

8. A cell matrix comprising:
a. a peptide derived from fibrinogen, consisting of a chain of amino acids having a sequence of KGSWYSMRKMSMKIRPFFPQQ, or a haptotactic fragment thereof;
b. a cell bound to the peptide; and
c. a structure for supporting the cell, said peptide being attached to the structure such that said cell is supported by said structure.

9. The cell matrix of claim 8, wherein the structure is a biomedical device.

10. The cell matrix of claim 9, wherein the biomedical device is prosthesis.

11. The cell matrix of claim 8, wherein the structure is a gel.

12. The cell matrix of claim 8, wherein the structure is a sheet of collagen.

13. A composition for assessing binding of a peptide to a receptor, comprising:
a. a peptide derived from fibrinogen, consisting of a chain of amino acids having a sequence of KGSWYSMRKMSMKIRPFFPQQ, or a haptotactic fragment thereof; and
b. a reponer for transmitting a signal, wherein the peptide is labelled with the reporter.

14. The composition of claim 13, wherein the reporter is selected from the group consisting of a fluorescent moiety and a radioactive moiety.

15. The composition of claim 13, wherein the receptor is a protein.

16. A method for determining binding of a peptide to a receptor, the method comprising the steps of:
a. providing the peptide labeled with a reporter for transmitting a signal, said peptide being a peptide derived from fibrinogen, consisting of a chain of amino acids having a sequence of KGSWYSMRKMSMKIRPFFPQQ, or a haptotactic fragment thereof;
b. contacting said peptide with the receptor; and
c. determining binding of said peptide to said receptor according to the signal transmitted by the reporter.

17. A method for separating a cell from a mixture, the cell being capable of binding to a peptide, the method comprising the steps of:
a. incubating the mixture with the peptide, said peptide being a peptide derived from fibrinogen, consisting of a chain of amino acids having a sequence of KGSWYSMRKMSMYIRPFFPQQ, or a haptotactic fragment thereof, such that the cell binds to the peptide; and
b. removing said peptide from said mixture, such that said cell is separated from said mixture.

18. The method of claim 17, wherein the peptide is attached to a support, such that the step of removing said peptide is performed by separating the support and the mixture.

19. The method of claim 18, wherein the support is a gel chromatography matrix.

20. A system for culturing of a cell capable of binding to a peptide, comprising:
a. a structure for attaching the peptide, the peptide being a peptide derived from fibrinogen, consisting of a chain of amino acids having a sequence of KGSWYSMRKMSMKIRPFFPQQ, or a haptotactic fragment thereof, such that the cell binds to the peptide and grows on said structure; and
b. a culture medium for contacting the cell to provide nourishment to said cell.

21. A polymer, comprising:
a. a plurality of subunits, each subunit featuring at least one peptide derived from fibrinogen, consisting of a chain of amino acids having a sequence of KGSWYSMRKMSMKIRPFFPQQ, or a haptotactic fragment thereof; and
b. a plurality of linker moieties for attaching each of the plurality of subunits to another of said plurality of subunits to form the polymer.

22. The polymer of claim 21, wherein the subunit is comprised of the at least one peptide, such that said polymer is a peptide polymer.

23. The polymer of claim 21, wherein the at least one peptide is attached to the subunit, such that said polymer is a co-polymer.

## Patentansprüche

1. Aus Fibrinogen gewonnenes Peptid, bestehend aus einer Kette von Aminosäuren mit einer Sequenz von KGSWYSMRKMSMKIRPFFPQQ oder einem haptotaktischen Fragment derselben.

2. Zusammensetzung, die ein aus Fibrinogen gewonnenes Peptid aufweist, welches aus einer Kette von Aminosäuren mit einer Sequenz von KGSWYSMRKMSMKIRPFFPQQ oder einem haptotaktischen Fragment derselben besteht, und die außerdem einen pharmazeutisch akzeptablen Träger aufweist.

3. Zusammensetzung nach Anspruch 2, die außerdem ein biologisches Agens aufweist, welches ausgewählt wird aus der Gruppe, die aus Arzneimitteln, Vitaminen, Vitaminderivaten, Wachstumsfaktoren, Glukokortikosteroiden, Steroiden, Antibiotika, Toxinen, Enzymen, Enzyminhibitoren, Immunmodulatoren, Immunglobulinen und Fragmenten von diesen, aus Fettsäurederivaten, Polysacchariden, Zellrezeptor-Bindungsmolekülen, entzündungshemmenden Agenzien, Nukleinsäuren und Polynukleotiden besteht.

4. Zusammensetzung nach Anspruch 2, die außerdem eine Zelle aufweist, welche ausgewählt wird aus der Gruppe, die aus Fibroblasten, Endothelialzellen, Chondrozyten, Neuroblastomzellen, Nierenzellen, Leberzellen, pankreatischen Zellen, Schilddrüsenzellen, Gliazellen, glatten Muskelzellen, Mammakarzinomzellen der Maus, knochenbildenden oder knorpelbildenden Zellen und Kombinationen hieraus besteht.

5. Zusammensetzung nach Anspruch 4, bei der der Zelltyp ausgewählt wird aus der Gruppe, die aus Fibroblasten, glatten Muskelzellen, Endothelialzellen, Chondrozyten und Kombinationen hieraus besteht.

6. Zusammensetzung nach Anspruch 5, bei der der Zelltyp ein Fibroblast ist.

7. Verwendung einer Zusammensetzung nach einem der Ansprüche 2-6 für die Herstellung eines Medikaments, mit dem die Heilung einer Wunde bei einem Patienten gefördert wird.

8. Zellmatrix, mit:
a) einem aus Fibrinogen gewonnenen Peptid, bestehend aus einer Kette von Aminosäuren mit einer Sequenz von KGSWYSMRKMSMKIRPFFPQQ oder einem haptotaktischen Fragment derselben;
b) einer mit dem Peptid verbundenen Zelle; und
c) einer Struktur zum Stützen der Zelle, wobei das Peptid so an der Struktur befestigt wird, dass die Zelle von der Struktur gestützt wird.

9. Zellmatrix nach Anspruch 8, bei der die Struktur eine biomedizinische Vorrichtung ist.

10. Zellmatrix nach Anspruch 9, bei der die biomedizinische Vorrichtung eine Prothese ist.

11. Zellmatrix nach Anspruch 8, bei der die Struktur ein Gel ist.

12. Zellmatrix nach Anspruch 8, bei der die Struktur eine Schicht aus Collagen ist.

13. Zusammensetzung zur Beurteilung einer Bindung eines Peptids an einen Rezeptor, mit:
a) einem aus Fibrinogen gewonnenen Peptid, bestehend aus einer Kette von Aminosäuren mit einer Sequenz von KGSWYSMRKMSMKIRPFFPQQ oder einem haptotaktischen Fragment derselben; und
b) einem Reporter zur Übertragung eines Signals, wobei das Peptid mit dem Reporter markiert ist.

14. Zusammensetzung nach Anspruch 13, bei der der Reporter ausgewählt wird aus der Gruppe, die aus einem fluoreszierenden Anteil und einem radioaktiven Anteil besteht.

15. Zusammensetzung nach Anspruch 13, bei der der Rezeptor ein Protein ist.

16. Verfahren zur Bestimmung der Bindung eines Peptids an einen Rezeptor, wobei das Verfahren folgende Schritte aufweist:
a) Bereitstellen des Peptids, das mit einem Reporter markiert ist, um ein Signal zu übertragen, wobei das Peptid ein aus Fibrinogen gewonnenes Peptid ist, das aus einer Kette von Aminosäuren mit einer Sequenz von KGSWYSMRKMSMKIRPFFPQQ oder einem haptotaktischen Fragment derselben besteht;
b) In-Kontakt-Bringen des Peptids mit dem Rezeptor; und
c) Bestimmen der Bindung des Peptids an den Rezeptor gemäß dem Signal, das von dem Reporter übermittelt wird.

17. Verfahren zum Separieren einer Zelle aus einer Mischung, wobei die Zelle in der Lage ist, sich an ein Peptid zu binden, wobei das Verfahren folgende Schritte aufweist:
a) Inkubieren der Mischung mit dem Peptid, wobei das Peptid ein aus Fibrinogen gewonnenes Peptid ist, das aus einer Kette von Aminosäuren mit einer Sequenz von KGSWYSMRKMSMKIRPFFPQQ oder einem haptotaktischen Fragment derselben besteht, so dass sich die Zelle an das Peptid bindet; und
b) Entfernen des Peptids aus der Mischung auf eine solche Weise, dass die Zelle aus der Mischung separiert wird.

18. Verfahren nach Anspruch 17, bei dem das Peptid so an einem Stützelement befestigt ist, dass der Schritt des Entfernens des Peptids ausgeführt wird, indem das Stützelement und die Mischung separiert werden.

19. Verfahren nach Anspruch 18, bei dem das Stützelement eine Gel-Chromatographiematrix ist.

20. System zur Bildung einer Kultur einer Zelle, die in der Lage ist, sich an ein Peptid zu binden, mit:
a) einer Struktur zur Befestigung des Peptids, wobei das Peptid ein aus Fibrinogen gewonnenes Peptid ist, das aus einer Kette von Aminosäuren mit einer Sequenz von KGSWYSMRKMSMKIRPFFPQQ oder einem haptotaktischen Fragment derselben besteht, so dass sich die Zelle an das Peptid bindet und auf der Struktur wächst; und
b) einem Kulturmedium, das mit der Zelle in Kontakt gebracht wird, damit die Zelle ernährt wird.

21. Polymer mit:
a) einer Vielzahl von Untereinheiten, wobei in jeder Untereinheit mindestens ein aus Fibrinogen gewonnenes Peptid vorkommt, das aus einer Kette von Aminosäuren mit einer Sequenz von KGSWYSMRKMSMKIRPFFPQQ oder einem haptotaktischen Fragment derselben besteht; und
b) einer Vielzahl von Verbindungsanteilen, um jede der mehreren Untereinheiten an einer anderen der mehreren Untereinheiten zu befestigen und damit das Polymer zu bilden.

22. Polymer nach Anspruch 21, bei dem die Untereinheit aus dem mindestens einen Peptid besteht, so dass das Polymer ein Peptidpolymer ist.

23. Polymer nach Anspruch 21, bei dem das mindestens eine Peptid an der Untereinheit befestigt ist, so dass das Polymer ein Copolymer ist.

## Revendications

1. Peptide dérivé d'un fibrinogène, constitué par une chaîne d'acides aminés ayant une séquence KGSWYSMRKMSMKIRPFFPQQ, ou un fragment haptotactique de celui-ci.

2. Composition, comprenant un peptide dérivé d'un fibrinogène constitué par une chaîne d'acides aminés ayant une séquence de KGSWYSMRKMSMKIRPFFPQQ, ou un fragment haptotactique de celui-ci, comprenant en outre, un porteur pharmaceutiquement acceptable.

3. Composition selon la revendication 2, comprenant en outre, un agent biologique choisis dans le groupe constitué par des médicaments, des vitamines, des dérivés de vitamines, des facteurs de croissance, des glucocorticostéroïdes, des stéroïdes, des antibiotiques, des toxines, des enzymes, des inhibiteurs d'enzyme, des immunomodulateurs, des immunoglobulines, et des fragments de celles-ci, des dérivés d'acides gras, des polysaccharides, des molécules de liaison aux récepteurs cellulaires, des agents anti-inflammatoires, des acides nucléiques, et des polynucléotides.

4. Composition selon la revendication 2, comprenant en outre, une cellule choisie dans le groupe des fibroblastes, des cellules endothéliales, des chondrocytes, des cellules de neuroblastome, des cellules rénales, des hépatocytes, des cellules du pancréas, des cellules thyroïdiennes, des cellules gliales, des cellules du muscle lisse, des cellules de carcinome mammaire de souris, des cellules formant les os ou du cartilage, et des combinaison de celles-ci.

5. Composition selon la revendication 4, dans laquelle le type de cellule est choisi dans le groupe comprenant des fibroblastes, des cellules de muscle lisse, des cellules endothéliales, des chondrocytes et des combinaisons de celles-ci.

6. Composition selon la revendication 5, dans laquelle le type de cellule est un fibroblaste.

7. Utilisation d'une composition selon l'une quelconque des revendications 2 à 6 pour la fabrication d'un médicament destiné à favoriser la cicatrisation d'une blessure chez un sujet.

8. Matrice cellulaire comprenant :
a. un peptide dérivé d'un fibrinogène, constitué par une chaîne d'acides aminés ayant une séquence KGSWYSMRKMSMKIRPFFPQQ ou un fragment haptotactique de celui-ci ;
b. une cellule liée au peptide ; et
c. Une structure pour supporter la cellule, ledit peptide étant attaché à la structure de telle sorte que ladite cellule est supportée par ladite structure.

9. Matrice cellulaire selon la revendication 8, dans laquelle la structure est un dispositif biomédical.

10. Matrice cellulaire selon la revendication 9, dans laquelle le dispositif biomédical est une prothèse.

11. Matrice cellulaire selon la revendication 8, dans laquelle la structure est un gel.

12. Matrice cellulaire selon la revendication 8, dans laquelle la structure est une feuille de collagène.

13. Composition pour évaluer la liaison d'un peptide à un récepteur, comprenant :
a. un peptide dérivé du d'un fibrinogène, constitué par une chaîne d'acides aminés ayant une séquence KGSWYSMRKMSMKIRPFFPQQ, ou un fragment haptotactique de celui-ci ; et
b. un reporteur pour transmettre un signal dans lequel le peptide est marqué avec le reporteur.

14. Composition selon la revendication 13, dans laquelle le reporteur est choisi dans le groupe comprenant un fragment fluorescent et un fragment radioactif.

15. Composition selon la revendication 13, dans laquelle le récepteur est une protéine.

16. Procédé permettant de déterminer la liaison d'un peptide à un récepteur, le procédé comprenant les étapes consistant à :
a. fournir le peptide marqué par un récepteur pour transmettre un signal, ledit peptide étant un peptide dérivé d'un fibrinogène, constitué par une chaîne d'acides aminés ayant une séquence KGSWYSMRKMSMKIRPFFPQQ, ou un fragment haptotactique de celui-ci ;
b. mettre en contact ledit peptide avec le récepteur ; et
c. déterminer la liaison dudit peptide audit récepteur selon le signal transmis par le reporteur.

17. Procédé permettant de séparer une cellule d'un mélange, la cellule étant capable de se lier à un peptide, le procédé comprenant les étapes consistants à :
a. mettre à incuber le mélange avec la peptide, ledit peptide étant un peptide dérivé d'un fibrinogène, constitué par une chaîne d'acides aminés ayant une séquence KGSWYSMRKMSMKIRPFFPQQ, ou un fragment haptotactique de celui-ci, de telle sorte que la cellule se lie au peptide ; et
b. retirer ledit peptide dudit mélange de telle sorte que ladite cellule est séparée dudit mélange.

18. Procédé selon la revendication 17, dans lequel le peptide est attaché à un support, de telle sorte que l'étape consistant à retirer ledit peptide est exécutée en séparant le support d'avec le mélange

19. Procédé selon la revendication 18, dans lequel le support est une matrice de chromatographie d'exclusion diffusion.

20. Système permettant de cultiver une cellule capable de se lier à un peptide, comprenant :
a. une structure pour attacher le peptide, le peptide étant un peptide dérivé d'un fibrinogène, constitué par une chaîne d'acides aminés ayant une séquence KGSWYSMRKMSMKIRPFFPQQ, ou un fragment haptotactique de celui-ci, de telle sorte que la cellule se lie au peptide et croisse sur ladite structure ; et
b. un milieu de culture pour mettre en contact la cellule afin de fournir une alimentation à ladite cellule.

21. Polymère, comprenant :
a. une pluralité de sous-unités, chaque sous-unité ayant pour caractéristique au moins un peptide dérivé d'un fibrinogène, constitué par une chaîne d'acides aminés ayant une séquence KGSWYSMRKMSMKIRPFFPQQ, ou un fragment haptotactique de celui-ci ; et
b. une pluralité de fractions de molécules de liaison pour attacher chacune de la pluralité de sous-unités à une autre de ladite pluralité de sous-unités pour former le polymère.

22. Polymère selon la revendication 21, dans lequel la sous-unité comprend le au moins un peptide de telle sorte que ledit polymère est un polymère peptidique.

23. Polymère selon la revendication 21, dans lequel le au moins un peptide est attaché à la sous-unité de telle sorte que ledit polymère est un copolymère.


### SEQUENCE LISTING

<110> HADASIT MEDICAL RESEARCH SERVICES & DEVELOPMENT LTD.
<120> NOVEL PEPTIDES
<130> 10223-7-NP
<150> US 09/084,371
   <151> 1998-05-27
<160> 4
<170> PatentIn version 3.0
<210> 1
   <211> 19
   <212> PRT
   <213> Artificial
<220>
   <223> Synthetic peptide
<400> 1
   Glu Ala Asp His Glu Gly Thr His Ser Thr Lys Arg Gly His Ala Lys
   1               5                   10                  15
   Ser Arg Pro
<210> 2
   <211> 21
   <212> PRT
   <213> Artificial
<220>
   <223> Synthetic peptide
<400> 2
   Lys Gly Ser Trp Tyr Ser Met Arg Lys Met Ser Met Lys Ile Arg Pro
   1               5                   10                  15
   Phe Phe Pro Gln Gln
               20
<210> 3
   <211> 20
   <212> PRT
   <213> Artificial
<220>
   <223> synthetic peptide
<400> 3
   Leu Thr Ile Gly Glu Gly Gln Gln His His Leu Gly Gly Ala Lys Gln
   1               5                   10                  15
   Ala Gly Asp Val
               20
<210> 4
   <211> 20
   <212> PRT
   <213> Artificial
<220>
   <223> synthetic peptide
<400> 4
   Arg Gly Ala Asp Tyr Ser Leu Arg Ala Val Arg Met Lys Ile Arg Pro
   1               5                   10                  15
   Leu Val Thr Gln
               20